# EUROPÄISCHE PATENTSCHRIFT

(11) **EP 0 963 737 B1**
(45) Veröffentlichungstag und Bekanntmachung des Hinweises auf die Patenterteilung: **27.08.2003**
(21) Anmeldenummer: 98108893.3
(22) Anmeldetag: 13.05.1998
(51) Int. Cl.: A61B 17/92

(54) **Chirurgische Nagelzange**
Pliers for surgical nails
Pince pour broches chirurgicales

(43) Veröffentlichungstag der Anmeldung: 15.12.1999
(73) Patentinhaber: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(56) Entgegenhaltungen:
- AT-B- 372 842
- CH-A- 687 122
- FR-A- 2 668 919
- FR-A- 2 743 490

## Beschreibung

Die Erfindung betrifft eine chirurgische Nagelzange.

Zum Eintreiben chirurgischer Nägel mit einem Schlagwerkzeug, beispielsweise einem Hammer, sind Einschlaginstrumente bekannt, welche die Nägel schlagund verliersicher halten *(FR-A-26 68 919, AT-B-372842)*. Zum Entfernen der Nägel sind aus offenkundiger Vorbenutzung Extraktionszangen bekannt, die in Aufbau und Funktion herkömmlichen Greifzangen entsprechen. Im operativen Einsatz ist die Verwendung vieler verschiedener Werkzeuge unter Handhabungsgesichtspunkten nachteilig. Je größer die Anzahl der verwendeten Werkzeuge, desto größer ist auch der Platzbedarf wie auch der Aufwand zur Bereitstellung und Reinigung. Auch ist die Notwendigkeit des Werkzeugwechsels bei rasch aufeinanderfolgendem Eintreiben und Extrahieren von Nägeln ungünstig, wenn beispielsweise ein gesetzter Nagel extrahiert und neu gesetzt werden muß, nachteilig.

Der Erfindung liegt die Aufgabe zugrunde, *eine chirurgische Nagelzange zu schaffen, bei der* die genannten Nachteile vermindert *sind.*

Die erfindungsgemäße Lösung liegt in den Merkmalen des Anspruchs 1, vorzugsweise auch in denen der Unteransprüche. Demgemäß ist bei einer medizinischen Nagelzange mit zwei gelenkig miteinander verbundenen Gliedern wenigstens eines der Glieder als ein Einschlagwerkzeug ausgebildet, bei dem zusätzlich zu einem gekrümmten Griffteil ein gestreckt ausgebildetes Verstärkungselement vorgesehen ist. Daraus ergibt sich, daß das als Einschlagwerkzeug ausgebildete Glied (im folgenden: Einschlagglied) trotz ergonomisch gekrümmter Form des Griffs zur Übertragung der Schlagkräfte auf den Nagel ausgebildet ist. Dies hat den Vorteil, daß ein und dasselbe Werkzeug zum Eintreiben und Extrahieren der Nägel zur Verfügung steht und ein Werkzeugwechsel nicht erforderlich ist. Abweichungen von der geradlinigen Streckung können mit Rücksicht auf das Gelenk zwischen den Zangengliedern in Kauf genommen werden.

Es ist eine gattungsfremde Anordnung zur Fixation mittels Klammern bekannt, die als Halteinstrument einen meißelartig ausgebildeten Schlagdorn mit einem daran drehbar angeordneten Schwenkhebel aufweist. Die Klammer ist in einer grabenartigen Vertiefung an der spitzen Seite des Schlagdorns gehalten und durch einen schnabelartigen Vorsprung des Schwenkhebels klemmend gesichert. Bei der bekannten Anordnung ist nachteilig, daß zum einen ein zweites Instrument zum vollständigen Einschlagen der Klammer erforderlich ist, da dies wegen des zwischen Klammer und Knochenoberfläche eingreifenden schnabelartigen Vorsprungs nicht mit dem Schlagdorn bewerkstelligt werden kann, und zum anderen der Schlagdorn zum Extrahieren nicht geeignet ist, da er bei vollständig eingeschlagener Klammer diese nicht greifen kann und sein gerader ausgeführter Griff nur die Übertragung geringer Zugkräfte erlaubt.

Das Verstärkungselement kann (aber muß nicht) einstückig mit idem Griff des Einschlagglieds ausgeführt sein. Damit die Schlagkräfte sicher auf den Nagel übertragen werden können, ist das Einschlagglied zweckmäßigerweise mit einer das hintere Ende des Nagels aufnehmenden Führung versehen, beispielsweise einer Bohrung. Das bedeutet nicht, daß nicht auch das andere Glied bei der Sicherung des Nagels in der Zange während des Eintreibens desselben beteiligt sein könnte. Je weniger dies erforderlich ist, um so besser ist es jedoch, damit der Operateur wenig Aufmerksamkeit auf das Halten der Zange zu verwenden braucht. Die Führung ermöglicht auch ein einfacheres Extrahieren, da erfahrungsgemäß ein Nagel durch die Aufbringung von Schwenkmomenten und Seitenkräften zusätzlich zu Zugkräften leichter zu lösen ist. Die Führung ist zweckmäßigerweise als Bohrung in dem Schnabelteil des Einschlagglieds ausgebildet. Die rückwärtige, der Öffnung gegenüberliegende Stirnfläche der Bohrung ist so ausgestaltet, daß sie zur Übertragung der Einschlagkräfte mit der hinteren Stirnfläche des Nagels zusammenwirkt. Zweckmäßigerweise ist am hinteren Ende des Einschlagglieds eine Schlagfläche angeordnet. Unter dem hinteren Ende wird das dem Schnabel abgewandte Ende des Griffes verstanden. Die zum Eintreiben des Nagels verwendete Schlagfläche ist als linke Stirnseite eines Schlagkopfes ausgebildet. Der Schlagkopf kann auch eine vordere Hammerschlagfläche aufweisen, die beim Extrahieren eines Nagels eingesetzt werden kann.

Zum Festhalten des Nagels in der Führung des Einschlagglieds wird zweckmäßigerweise das andere Zangenglied herangezogen, das deshalb im folgenden auch als Halteglied bezeichnet wird.

Wenn die Führung für das hintere Nagelende als Bohrung ausgebildet ist, weist diese vorteilhafterweise eine seitliche, d.h. quer zu ihrer Längsachse gelegene Öffnung auf, die dem Halteglied zugewandt ist und durch die ein an dem Halteglied angeordneter Haltevorsprung eingreifen kann, um *bei* geschlossener Zange haltend auf den Nagel einzuwirken. Das kann durch Kraftschluß geschehen, *jedoch* wirkt vorteilhafterweise der Vorsprung im Formschluß mit dem Nagel zusammen. Dazu ist es zweckmäßig, wenn der Nagel in seinem hinteren Schaftbereich einen mit dem Haltevorsprung zusammenwirkenden Rezeß aufweist.

Nach einem weiteren vorteilhaften Merkmal der Erfindung *weist* die Zange Zuggriffe, d.h. quer zur Extraktionsrichtung angeordnete Griffteile, auf. Zur Aufbringung der zum Extrahieren benötigten Zugkraft ist es gleichgültig, ob die Zuggriffe weiter vorne oder hinten an dem Zangengriff angeordnet sind; jedoch ist es günstig, wenn sie im vorderen Bereich der Griffe angeordnet sind, damit der Chirurg sie bei der Extraktion mit den Fingern greifen und gleichzeitig mit der Handfläche den Zangengriff umschließen kann.

Die erfindungsgemäße Nagelzange wird nachfolgend unter Bezugnahme auf die Zeichnung, die ein vorteilhaftes Ausführungsbeispiel darstellt, erläutert. Es zeigen:

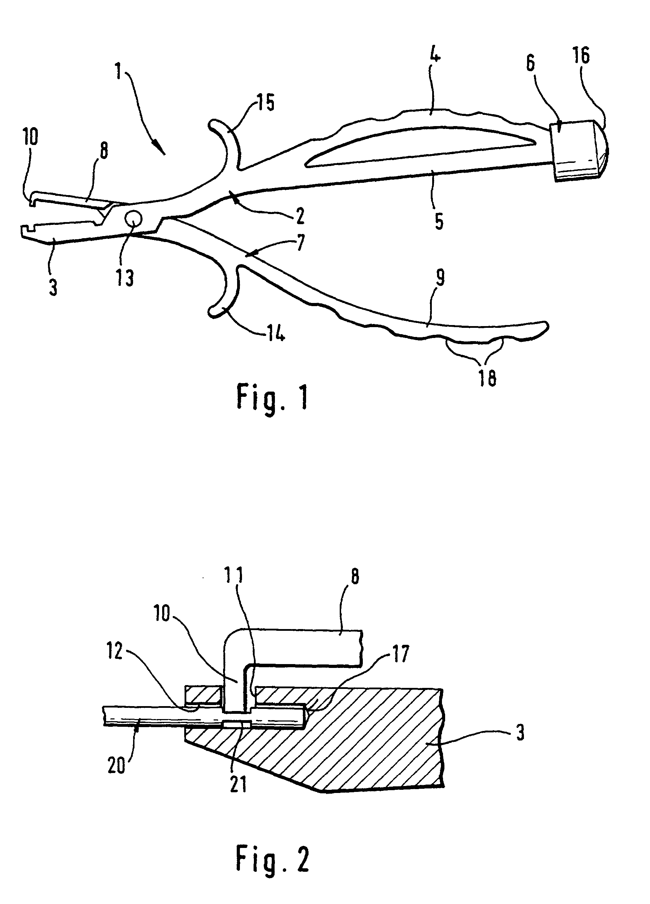

- Fig. 1: die Nagelzange in einer geöffneten Stellung und
- Fig. 2: einen Längsschnitt durch den Schnabel der Zange mit eingesetztem Nagel in einer vergrößerten Darstellung.

Die chirurgische Nagelzange 1 besteht aus zwei über ein Drehgelenk 13 miteinander verbundene Gliedern 2, 7, die je einen Griff 4, 9 und einen Schnabelteil 3, 8 aufweisen. Vorzugsweise ist sie aus einem korrosionsbeständigen Material, wie nichtrostendem Stahl, gefertigt.

Das Zangenglied 2, das das Einschlagglied bildet, weist in seinem Schnabelteil 3 eine längs angeordnete Bohrung 12 zur Aufnahme des hinteren Ende des Nagels 20 auf. Deren Durchmesser ist so gewählt, daß sich eine Spielpassung mit dem Nagel 20 ergibt. Die Bohrung 12 erstreckt sich als Sackloch über eine Länge, die für eine sichere Führung des Nagels 20 ausreicht, beispielsweise in der Größenordnung von einem Zentimeter. Sie weist eine dem anderen Schnabelteil 8 zugewandte seitliche Öffnung 11 auf. An dem vorderen Ende des Schnabelteils 8 ist ein Haltevorsprung 10 angeordnet, der bei geschlossener Zange in die Öffnung 11 eingreift. Die Öffnung 11 muß nicht, aber darf, deutlich größer als der Haltevorsprung 10 sein. Wichtig ist, daß der Haltevorsprung 10 durch die Öffnung 11 hindurch mit dem Nagel 20 zusammenwirkt, so daß der Nagel 20 bei geschlossener Zange 1 sicher geklemmt ist. Um zum Extrahieren Formschluß zu gewähren, ist ein Rezeß 21 so an dem Nagel 20 angeordnet, daß der Haltevorsprung 10 in den Rezeß 21 eingreifen kann. Die Stirnfläche 17 der Bohrung 12 dient im allgemeinen zur Übertragung der Schlagkräfte auf den Nagel 20.

Die Griffe 4, 9 sind von einer ergonomisch bogenförmigen Gestalt. An ihren Außenseiten sind sie mit mehreren Griffmulden 18 versehen. An dem hinteren Ende des Griffes 4 des Einschlagglieds 2 ist ein Schlagkopf 6 vorgesehen, der eine quer zu der Achse der Bohrung 12 ausgerichtete Schlagfläche 16 mit ausgeprägter Balligkeit aufweist. Die Achse der Bohrung 12 ist auf die Schlagfläche ausgerichtet.

An dem Griff 4 ist sehnenartig ein Verstärkungselement 5 vorgesehen, dessen hinteres Ende etwa mittig an der der Schlagfläche 16 abgewandten Seite des Schlagkopfes 6 angeordnet ist und dessen vorderen Ende nahe dem Gelenk 13 mit dem Griff 4 einstückig verbunden ist. Das Verstärkungselement 5 ist geradlinig ausgeführt und ausreichend stark bemessen, um alle praktisch vorkommenden Schlagkräfte sicher und ohne Ausknikken zu übertragen. Es verläuft nahezu in der Flucht zur Bohrung 12. Seine Abweichung von der geraden Fluchtlinie ist in dem dargestellten Beispiel zur Schaffung von hinreichendem Zwischenraum zwischen den Zangengriffen für die Hand des Chirurgen vorgesehen. Gleichwohl ist der Verlauf des Verstärkungselementes und der bis zum Schnabelende anschließenden Teile des Einschlagglieds weitgehend senkrecht, so daß das Einschlagglied nur wenig von der Wirkungslinie der Schlagkraft abweicht und nicht zu befürchten ist, daß es unter der Schlagwirkung zur Seite ausweicht.

In dem gelenknahen Bereich der Griffe 4, 9 sind einander gegenüberliegend zwei bogenförmige Zuggriffe 14, 15 angeordnet. Sie liegen in der durch die Griffe 4, 9 definierten Ebene und weisen jeweils etwa senkrecht von ihnen weg. Sie sind zur Aufnahme je eines Fingers bei der Extraktion von Nägeln bestimmt. Der Anbringungsort der Zuggriffe 14, 15 ist so gewählt, daß, wenn sie beispielsweise von Zeige- und Mittelfinger einer Hand ergriffen werden, die Griffe 4, 5 in der Handfläche zu liegen kommen. Auf diese Weise können bei sicherer Führung der Zange hohe Zugkräfte aufgebracht werden, zusätzlich können Dreh- und Schwenkbewegungen ausgeübt werden.

## Patentansprüche

1. Chirurgische Nagelzange, **dadurch gekennzeichnet, daß** wenigstens eines ihrer Glieder (2, 7) als Einschlagwerkzeug ausgebildet ist, das zusätzlich zu einem gekrümmten Griffteil (4) ein gestreckt ausgebildetes Verstärkungselement (5) aufweist.

2. Nagelzange nach Anspruch 1, **dadurch gekennzeichnet, daß** das Einschlagglied (2) der Zange an dem Ende seines Schnabelteils (3) eine Längsführung für das hintere Ende eines Nagels (20) aufweist.

3. Nagelzange nach Anspruch 1 oder 2, **dadurch gekennzeichnet, daß** das Einschlagglied (2) an seinem hinteren Ende eine Schlagfläche (16) bildet.

4. Nagelzange nach einem der -Ansprüche 4 *1 bis 3*, **dadurch gekennzeichnet, daß** das Verstärkungselement einstückig mit dem Einschlagglied (2) ausgeführt ist.

5. Nagelzange nach Anspruch 2, **dadurch gekennzeichnet, daß** die Führung als Bohrung (12) ausgebildet ist.

6. Nagelzange nach Anspruch 5, **dadurch gekennzeichnet, daß** die Bohrung (12) eine seitliche Öffnung (11) aufweist und das andere Zangenglied (7) einen darin eingreifenden Haltevorsprung (10) aufweist.

7. Nagelzange nach einem der Ansprüche 1 bis 7 6, **dadurch gekennzeichnet, daß** die Zange Zuggriffe aufweist.

8. Anordnung aus einer Zange nach Anspruch 1 und einem Nagel, der nahe seinem hinteren Ende einen Rezeß aufweist, **dadurch gekennzeichnet, daß** die seitliche Öffnung (11) und der Haltevorsprung (10) bei in die Bohrung (12) bis zum Anschlag eingesetzten Nagel (20) mit dessen Rezeß haltend zusammenwirkt.

## Claims

1. A pair of surgical pliers, **characterised in that** at least one of its members (2,7) is in the form of a driving-in tool which, in addition to a curved handle part (4), has an elongate reinforcing element (5).

2. A pair of pliers according to Claim 1, **characterised in that** the driving-in member (2) of the pliers has at the end of its jaw part (3) a longitudinal guide for the rear end of a pin (20).

3. A pair of pliers according to Claim 1 or 2, **characterised in that** the driving-in member (2) forms at its rear end an impact surface (16).

4. A pair of pliers according to any one of Claims 1 to 3, **characterised in that** the reinforcing element is formed in one piece with the driving-in member (2).

5. A pair of pliers according to Claim 2, **characterised in that** the guide is in the form of a bore (12).

6. A pair of pliers according to Claim 5, **characterised in that** the bore (12) has a lateral opening (11) and the other pliers part (7) has a retaining projection (10) engaging therein.

7. A pair of pliers according to any one of Claims 1 to 6, **characterised in that** the pliers have pull grips.

8. An assembly of a pair of pliers according to Claim 1 and a pin which has a recess near to its rear end, **characterised in that** the lateral opening (11) and the retaining projection (10) co-operate retentively with the recess in the pin (20) when the latter is inserted into the bore (12) until it abuts.

## Revendications

1. Tenailles chirurgicales, **caractérisées en ce qu'**au moins l'un de leurs éléments (2, 7) est constitué en tant qu'outil de percussion qui, en plus d'une partie (4) en poignée courbée, présente un élément (5) de renforcement de forme droite.

2. Tenailles chirurgicales selon la revendication 1, **caractérisées en ce que** l'élément (2) de percussion de la tenaille, à l'extrémité de sa partie (3) de bec, présente un guidage en longueur pour l'extrémité arrière d'un clou (20).

3. Tenailles chirurgicales selon la revendication 1 ou 2, **caractérisées en ce que** l'élément (2) de percussion forme au niveau de son extrémité arrière une surface (16) de frappe.

4. Tenailles chirurgicales selon l'une quelconque des revendications là 3, **caractérisées en ce que** l'élément de renforcement est réalisé d'un seul tenant avec l'élément (2) de percussion.

5. Tenailles chirurgicales selon la revendication 2, **caractérisées en ce que** le guidage est constitué d'un perçage (12).

6. Tenailles chirurgicales selon la revendication 5, **caractérisées en ce que** le perçage (12) présente une ouverture (11) latérale et que l'autre élément (7) de tenailles présente une saillie (10) de maintien qui s'y engage.

7. Tenailles chirurgicales selon l'une quelconque des revendications 1 à 6, **caractérisées en ce que** les tenailles présentent des branches de tirage.

8. Disposition de tenailles selon la revendication 1 et d'un clou qui, à proximité de son extrémité arrière présente un creux **caractérisée en ce que** l'ouverture (11) latérale et la saillie (10) de maintien agissent ensemble avec ce creux de façon à réaliser un maintien du clou (20) introduit dans le perçage (12) jusqu'à buter.
